# EUROPEAN PATENT SPECIFICATION

(11) **EP 4 426 401 B1**
(45) Date of publication and mention of the grant of the patent: **21.01.2026**
(21) Application number: 22826481.8
(22) Date of filing: 04.11.2022
(51) Int. Cl.: A61M 15/00, A61M 11/00

(54) **COMPRESSED GAS NEBULIZER**
DRUCKGASZERSTÄUBER
NÉBULISEUR À GAZ COMPRIMÉ

(30) Priority: 05.11.2021 US 202163276489 P
(43) Date of publication of application: 11.09.2024
(73) Proprietor: SunMed Group Holdings, LLC, Grand Rapids, MI 49544 (US)
(72) Inventor: PIERRO, Brian William, Mettawa, IL 60045 (US); MENDOZA, Carlos Aceves, Mettawa, IL 60045 (US); FLEMING, Daniel William, Mettawa, IL 60045 (US)
(74) Representative: Wallinger Ricker Schlotter Tostmann
(86) International application number: PCT/US2022/049038
(87) International publication number: WO 2023/081411

(56) References cited:
- GB-A- 2 536 259
- US-A1- 2012 132 199
- US-A1- 2013 096 493
- US-A1- 2018 344 950
- US-A1- 2020 121 871

## Description

### CROSS-REFERENCE TO RELATED APPLICATIONS

This application claims the benefit of U.S. Provisional Application No. 63/276,489, filed on November 5, 2021,.

### TECHNICAL FIELD

The present disclosure generally relates to nebulizers for dispensing aerosolized medication to a patient, and particularly to nebulizers that operate by a compressed gas. US 2018/344950 A1 describes a medication delivery device adapted for delivery of a precise quantity of medication to mucosal tissue of a subject comprising: a gas-tight housing comprising a proximal and a distal end, said distal end comprising a conduit through which medication is discharged; a trigger mechanism incorporated into the proximal end of said housing wherein on actuation, a pressurized gas canister releases pressurized gas within said gas-tight housing; a pneumatic actuator comprising a proximal end which forms a gas-tight seal within said gas-tight housing, and a distal end terminating in a shaft leading to a piston, which, on exposure to said pressurized gas within said gas-tight housing, is induced to travel toward the distal end of said gas-tight housing; a piston, either unitary with or juxtaposed to the distal end of said pneumatic actuator and which is free to move toward the distal end of said housing through a bore, upon being impelled to move by said pneumatic actuator; a sealed medication storage depot comprising a precise quantity of a medication, wherein said storage depot is defined at its proximal aspect by said piston, on either side by the walls of said storage depot within a bore through which said piston travels on being impelled by said pneumatic actuator, and by a rupture-able disc at its distal aspect; a conduit adapted for mucosal delivery of medication through which medication is discharged upon said rupture-able disc being ruptured due to pressure from said piston driving said medication through said medication storage depot into said conduit for discharge at the distal end of said housing. US 2012/132199 A1 describes a nebuliser for nebulising a liquid medicament preparation, with a conveying device in order to presssurise the liquid and nebulise the liquid through a discharge nozzle, characterised in that the conveying device comprises a piston displaceable in a cylinder and an associated roll or hose seal to seal between the cylinder and the piston, and/or that the conveying device comprises or forms a pump that can be driven by a hydraulic or pneumatic working medium, so as to pressurise the liquid and nebulise the liquid through the discharge nozzle, or that the conveying device can be driven with a hydraulic or pneumatic working medium and comprises a pressure booster so as to raise the pressure acting on the liquid compared to the pressure of the working medium. US 2020/121871 A1 describes a beneficial agent dispensing device, comprising: at least one primary container storing a beneficial agent; a dispenser body associated with the primary container and comprising a pressure chamber and at least one dispensing port, wherein activation of said pressure chamber causes said primary container to flex and expel the beneficial agent through the dispensing port. GB 2 536 259 A describes a device for atomising a liquid, the device comprising a housing comprising a reservoir of liquid fluidly connected to a nebulising nozzle, a sealed space containing a cylinder of pressurised actuating gas, and a moveable barrier which separates the reservoir from the sealed space, wherein the device is operable in use to allow the pressurised actuating gas to escape from the cylinder into the sealed space and act on the moveable barrier to force liquid in the reservoir to pass through the nozzle and be emitted as an aerosol.

### SUMMARY

The The invention is defined in the independent claims 1 and 11. Preferred embodiments are matter of the dependent claims. present disclosure provides a compressed gas nebulizer that can include a power source portion that is configured to provide energy for causing the aerosolization of a medication, and an aerosol generation portion that is configured to receive the energy from the power source portion to aerosolize medication and move the aerosolized medication toward an opening of the device.

A pressurized gas nebulizer according to the present disclosure can comprise a nebulizer housing comprising a first end and a second end; a cartridge containing pressurized gas coupled to the nebulizer housing; a linear actuator comprising a series of linear positioned abutments, the linear actuator configured to be movable along the nebulizer housing; a trigger configured to engage one or more abutments as the linear actuator moves along the nebulizer housing; a medication chamber configured to contain medication to be dispensed from the first or second end of the nebulizer housing; and a nozzle, through which the medication is aerosolized before the medication is dispensed from the nebulizer housing; wherein when pressurized gas is conveyed from the cartridge, pressure within the nebulizer housing from the pressurized gas forces the linear actuator to move within the nebulizer housing. The aerosolized medication is dispensed from the nebulizer housing through a mouthpiece that can include baffles to direct entrained air to mix with the aerosolized medication.

The pressurized gas nebulizer may provide that advancement of the linear actuator is sequentially stopped at each respective abutment. Further, pressurized gas within the cartridge may not be released from the cartridge until the cartridge is punctured. In some embodiments, the cartridge is punctured by rotation of a housing containing the cartridge. Some embodiments further include a lock on the trigger to restrict actuation of the trigger until the trigger is unlocked.

It is understood that other configurations of the subject technology will become readily apparent to those skilled in the art from the following detailed description, wherein various configurations of the subject technology are shown and described by way of illustration. As will be realized, the subject technology is capable of other and different configurations and its several details are capable of modification in various other respects, all without departing from the scope of the subject technology. Accordingly, the drawings and detailed description are to be regarded as illustrative in nature and not as restrictive.

### BRIEF DESCRIPTION OF THE DRAWINGS

The following figures are included to illustrate certain aspects of the embodiments and should not be viewed as exclusive embodiments. The subject matter disclosed is capable of considerable modifications, alterations, combinations, and equivalents in form and function, as will occur to those skilled in the art and having the benefit of this disclosure.
FIG. 1A illustrates a perspective view of embodiments of a compressed gas nebulizer.
FIG. 1B illustrates a partially transparent perspective view of embodiments of a compressed gas nebulizer.
FIG. 2A illustrates a perspective view of embodiments of a compressed gas nebulizer.
FIG. 2B illustrates a close-up view of embodiments of a compressed gas nebulizer.
FIG. 3A illustrates a side view of embodiments of a portion of a compressed gas nebulizer.
FIG. 3B illustrates a partial cross-sectional view of embodiments of a portion of a compressed gas nebulizer.
FIG. 4A illustrates a side view of embodiments of a portion of a compressed gas nebulizer.
FIG. 4B illustrates a partial cross-sectional view of embodiments of a portion of a compressed gas nebulizer.
FIG. 5A illustrates a partially transparent perspective view of embodiments of a compressed gas nebulizer.
FIG. 5B illustrates a close-up view of embodiments of a compressed gas nebulizer.
FIG. 5C illustrates another close-up view of embodiments of a compressed gas nebulizer.
FIG. 6A illustrates a partially transparent perspective view of embodiments of a compressed gas nebulizer.
FIG. 6B illustrates a partial cross-sectional view of embodiments of a portion of a compressed gas nebulizer.

### DETAILED DESCRIPTION

The detailed description set forth below describes various configurations of the subject technology and is not intended to represent the only configurations in which the subject technology may be practiced. The detailed description includes specific details for the purpose of providing a thorough understanding of the subject technology. Accordingly, dimensions may be provided in regard to certain aspects as non-limiting examples. However, it will be apparent to those skilled in the art that the subject technology may be practiced without these specific details. In some instances, well-known structures and components are shown in block diagram form in order to avoid obscuring the concepts of the subject technology.

It is to be understood that the present disclosure includes examples of the subject technology and does not limit the scope of the appended claims. Various aspects of the subject technology will now be disclosed according to particular but non-limiting examples. Various embodiments described in the present disclosure may be carried out in different ways and variations, and in accordance with a desired application or implementation.

Nebulizers for providing aerosolized medicine to a patient are commonly used in the healthcare industry. In some instances, it is desirable that the nebulizer provides delivery to the patient by using a compressed gas that is contained in the nebulizer itself. This provides versatility in the settings that the nebulizer can be used in and the conditions for its use. For example, a compressed gas nebulizer device may be used by healthcare professionals in institutional settings or solely by the patient in nearly any location (e.g., home, ambulatory, traveling, planes, outdoors, etc.) and under extreme environmental conditions (e.g., -20°C to 60°C, sea level to 15,000 feet, airliners, etc.). The patient population for self-delivery ranges from pediatric to geriatric, and the compressed gas nebulizer device may also be administered by healthcare professionals/trained parents to neonatal and infant patient populations (e.g., surfactant delivery). Applications may include, but are not limited to, delivery of bronchodilators, corticosteroids, asthma medications, antibiotics/antivirals, and/or surfactants to applicable patient populations.

Described herein are embodiments of compressed gas nebulizers that provide aerosolized medication delivery to patients. In some embodiments, the aerosol generation and delivery mechanism are based solely on mechanical and pneumatic drivers and does not require electrical or battery power. In some aspects of the present disclosure, the energy source or catalyst for aerosol generation and delivery can be compressed gas. The compressed gas can provide readily available energy for causing medication to become aerosolized, relative other sources of energy, such as electricity, a compressor, or a mechanical driver. To provide a readily available source of energy, the compressed gas can be provided from a compressed gas cylinder coupled to the nebulizer. This provides the advantage of reliable medication delivery nearly anywhere and anytime and having a long shelf life. The ability to accurately control the rate of gas flow and, thus the medication output rate, enables optimization of the dose delivery based on the medication and patient needs.

The described embodiments provide nozzles that create aerosols in targeted median aerodynamic diameter ("MMAD") and geometric standard deviations ("GSD"). The nozzles are used with high pressure gas sources (up to about 20 bar) that force a liquid to be aerosolized through a filter and micro-nozzles to produce an aerosol plume, where embodiments of the present disclosure us compressed gas, such as a compressed gas cartridge, as the high pressure gas sources. The features and functionality of the present disclosure do not require ultrasonics, electrical power, or mechanical work to create the high pressure, and do not induce heat that could degrade the medication, yet it provides a robust single-patient use aerosol medication delivery system that can be used as a rescue device or for maintenance delivery of a wide range of respiratory medications.

In operation as a rescue device, embodiments described herein could be single-use and disposable. Once the device is activated, it can produce a single dose or multiple doses within a reasonable time duration (e.g., 5 to 30 inhalation maneuvers over a period within 24 hours of activation, based on the specific therapeutic needs and recommended dosing of the specific medication).

The rescue device is preferably small and compact, having the same or similar size range of an epi-pen. It is preferably pre-loaded with specific liquid medication(s). Many liquid respiratory medications can be packaged in about 3ml to about 10ml cartridges, including but not limited to, bronchodilators, corticosteroids, asthma medications, or surfactants (surfactants may be a larger volume and/or specialty device). The medication cartridges can be color coded and/or bar/QR coded for easy identification and tracing.

The device may be re-loaded with a compressed gas cylinder or cartridge that would provide the energy and inert gas to force the liquid medication through the nozzle(s) to produce an aerosol plume in the desired range of about 2-5 microns (or other targets based on the medication). These cylinders can be charged at the relatively high pressures (~4 times typical medical gas wall sources) used with the nozzles to produce a quality aerosol while a metering mechanism provides risk mitigations to protect the end-user from the relatively high pressure.

The device is capable of delivering multiple doses, defined as a slow, deep inhalation cycle, starting an inhalation cycle and firing at a desired inspiratory gas flow rate (or inspiratory effort). The number of doses per device would be commensurate with the clinical needs of medicinal substance (i.e., a function of the specific liquid medication). The device would produce an aerosol plume for a period of time commensurate with the medication being delivered and potentially more than an individual breath. The intent and benefit of the specific duration that is greater than a single breath is that it may eliminate or reduce the sensitivity to aerosol plume timing and coordination.

The aerosol output rate may be targeted, or adjustable, for specific medications/applications. The device, before use, is robust and self-contained, requiring no electrical power source, and remains operational in a wide range of environmental conditions and after typical or extreme abuse (e.g., storage in purses, pockets, cars, backpacks, altitudes including flight, drops, -20°C to 60°C) and a life expectancy/shelf-life of 5 years or more.

In some applications, the initial activation (from completely robust to ready-to-use) can be triggered by a mechanical action (e.g., removal of cap, flip trigger), and then the device is ready to fire upon activation. Because of its simplicity and design, the device is intuitive to use for a wide range of pediatric to geriatric patients and indicates when it is depleted. After activation and use, the device may be disposed of by recycling and/or discarded without special requirements because of the device materials and design.

In some applications, the device described herein may be a maintenance device that is a multi-use device that utilizes replaceable medication cartridges and gas cylinders (single-patient, multiple use). The same benefits and features for the rescue device are applicable for the maintenance device, and the maintenance device provides for replacement of the liquid medication cartridge and gas cylinder. These applications are designed for portable maintenance treatments and are options if a rescue/single use device is not advantageous.

Some applications provide a non-invasive surfactant delivery system for neonatal patients. Surfactants are delivered to newborns that have surfactant deficiencies, particularly patients that are 28 weeks, or less, of gestation. In some instances, surfactants are able to be efficiently and effectively delivered via aerosol (e.g., non-invasively), and these fragile patients can be treated with less invasive therapies and reduce the risks associated with invasive techniques and invasive ventilation. Additionally, the devices described herein can be used with non-invasive respiratory devices, such as nCPAP (nasal Continuous Positive Pressure) or HFNC (High Flow Nasal Cannula).

Described herein are embodiments of a nebulizer device powered by a highpressure pneumatic cartridge filled with a relevant volume of inert gas (e.g., medical air, CO2, or Nitrogen) at a pressure high enough to drive liquid medication through a nozzle at a rate and duration that is controlled by a linear actuator when a trigger is activated.

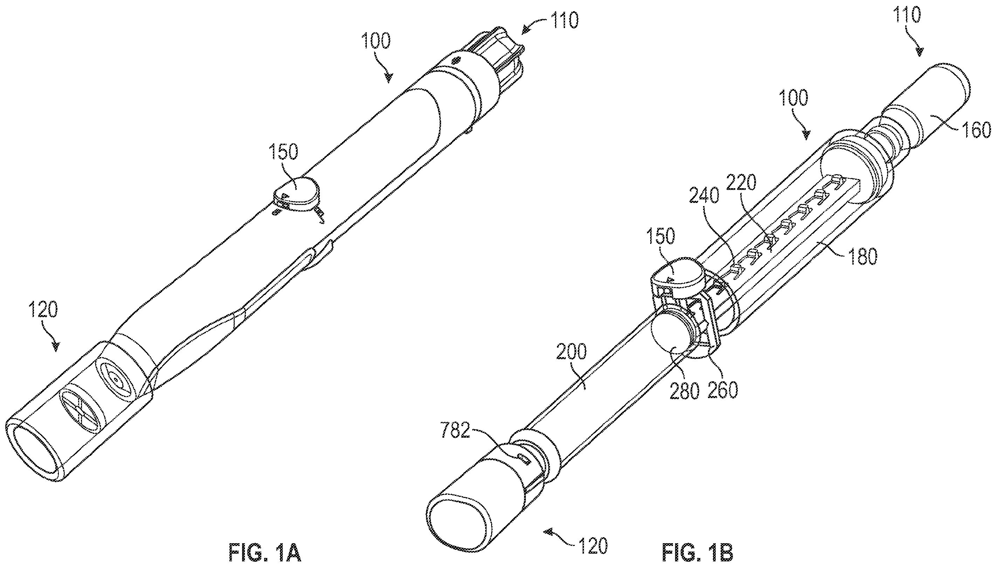

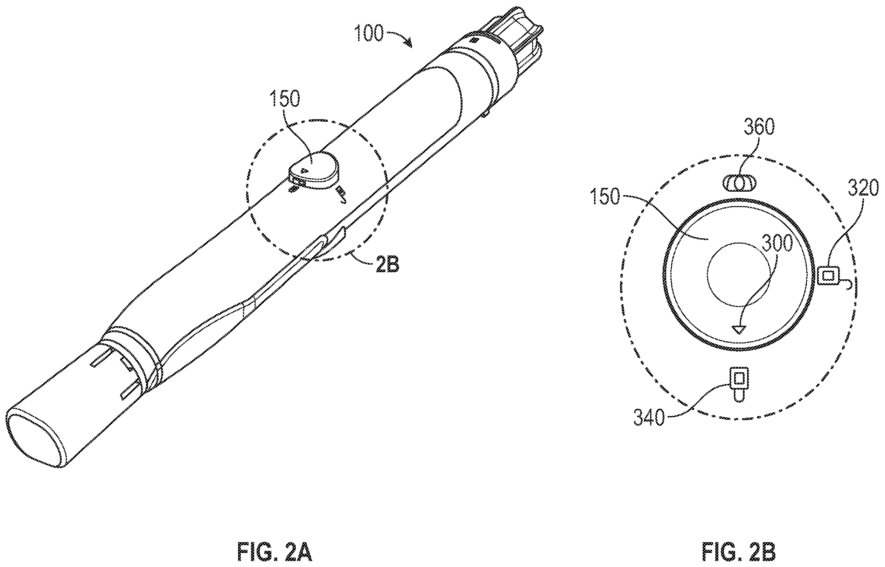

With reference to Figure 1A, a nebulizer 100 is illustrated having a first end 110 and a second end 120. The nebulizer 100 is activated by a trigger cap 150. As illustrated in Figure 2B, the nebulizer 100 includes a pneumatic cannister or cartridge 160 at the first end 110. Adjacent to the cartridge 160 is a high pressure chamber 180, and a medication chamber 200 is positioned between the high pressure chamber 180 and the second end 120. The nebulizer 100 includes a linear actuator 220 positioned within the high pressure chamber 180. Along the linear actuator 220 are a series of linear positioned abutments or actuator notches 240 that engage a trigger actuator 260, coupled to the trigger cap 150. The linear actuator 220 drives a plunger 280 through the medication chamber 200.

Figures 2A and 2B illustrate embodiments of the nebulizer 100 and the trigger cap 150. As illustrated in Figure 2B, the trigger cap 150 can include a locking indicator 300 that, when rotated, will identify whether the trigger cap 150 is in an unlocked position 320 or a locked position 340. When in the locked position 340, the trigger cap will not be able to be depressed to actuate the nebulizer 100. To actuate the nebulizer 100, the trigger cap 150 is rotated from the locked position 340 to the unlocked position 320. Thereafter, the trigger cap 150 can be depressed to actuate the nebulizer 100. Adjacent the trigger cap 150, a dose window 360 can identify either the number of doses used or the number of doses remaining.

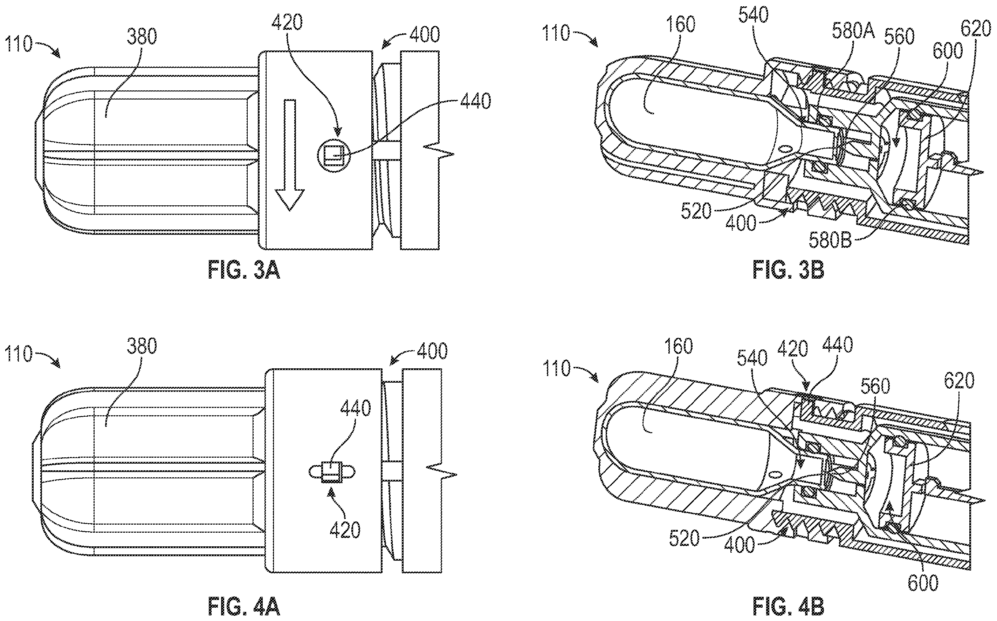

Figures 3A and 3B illustrate the first end 110 of the nebulizer 100 and provide a more detailed illustration of the cartridge 160 and its operation. As illustrated in Figure 3A, a cartridge cover 380 is provided to house the cartridge 160. The cartridge cover 380 is rotatably coupled to the nebulizer 100 via threads 400. The cartridge cover 380 preferably includes one or more windows 420 to communicate to the user proper rotation of the cartridge cover 380 to pierce the cartridge 160 and to arm the nebulizer 100. Visible through the window 420 is a tab 440 that can guide movement of the cartridge cover 380 or provide an indication that the cartridge cover 380 has been sufficiently rotated and the nebulizer 100 is armed.

With reference to Figure 3B, the cartridge 160 is illustrated to have a safety cap 520 located at a cartridge release end 540 of the cartridge 160. The cartridge 160 is coupled to the remainder of the nebulizer 100 adjacent a spike 560 that is configured to pierce the safety cap 520 when the cartridge cover 380 is rotated along the threads 400, and the cartridge is advanced toward the spike 560. Prior to rotation of the cartridge cover 380, the spike 560 does not pierce the safety cap 520 and the pressurized gas within the cartridge is contained only within the cartridge. When the cartridge cover 380 is rotated along the threads 400 (or the cartridge cover 380 is otherwise advanced toward the spike 560), the cartridge cover 380 carries and advances the cartridge 160 toward the spike 560.

The safety cap 520 of the cartridge 160 is positioned between first and second seals 580A, 580B, forming a first chamber 600 between the first and second seals 580A, 580B, the safety cap 510, and a piston 620, which is coupled to the linear actuator 220. Before the spike 560 pierces the safety cap 520 of the cartridge 160, the pressure within the first chamber 600 is not sufficient to drive the piston 620 or move the linear actuator 220.

In some embodiments of the present disclosure, the first chamber 600 is formed by the first seal 580A positioned between the housing and a cartridge 160 coupled thereto, and the second seal 580B positioned between the housing and the piston 620.

As illustrated in Figures 4A and 4B, when the cartridge cover 380 is rotated, or otherwise advanced, and the safety cap is pierced by the spike 560, pressurized gas is communicated from the cartridge 160 to the first chamber 600, increasing the pressure within the first chamber 600. In some embodiments, the cartridge cover 380 may be removed and the cartridge 160 may be manually advanced by applying a direct force or strike. This would allow the nebulizer to be used in emergency situations. In further embodiments, the cartridge cover 380 may be rotated from a locked position to an unlocked position that will permit manual advancement of the cartridge by pressing or striking the cartridge cover 380. This embodiment also provides functionality for emergency circumstances. When the safety cap is pierced, the pressurized gas increases the pressure in the first chamber 600, and the increased pressure presses against the piston 620 and the linear actuator 220.

The cartridge 160 may be precisely pre-charged at a pressure and volume that may be universal for all medication applications or specifically charged to meet the needs of a specific liquid medication. The cartridge 160 is preferably manufactured and installed in a safe, self-contained form that can handle the rigors of a wide range of environments (extreme heat, cold, altitude, etc.) and rough handling.

The volume of the high pressure chamber 180 is important such that the loss of volume associated with each single dose does not substantially alter, or reduce, the pressure exerted on the liquid medication over the course of all doses (e.g., up to 30 per device).

Once the cartridge 160 is pierced, and the first chamber 600 is pressurized, the nebulizer 100 is activated and ready to deliver one or more doses of aerosolized medication.

In some embodiments, the nebulizer 100 can provide up to approximately 30 doses of aerosolized medication. Each single dose may be initiated by pressing the trigger cap 150. The dose volume and dose duration are optimized for the specific medication in the liquid medication vial (or the medication chamber 200) by the interaction of the trigger actuator 260 and the linear actuator 220. The drive pressure within the first chamber 600 and the high pressure chamber 180 forces the liquid medication through a nozzle or plurality of nozzles, which may also contain an integral filter to protect the nozzle(s) from clogging due to contamination.

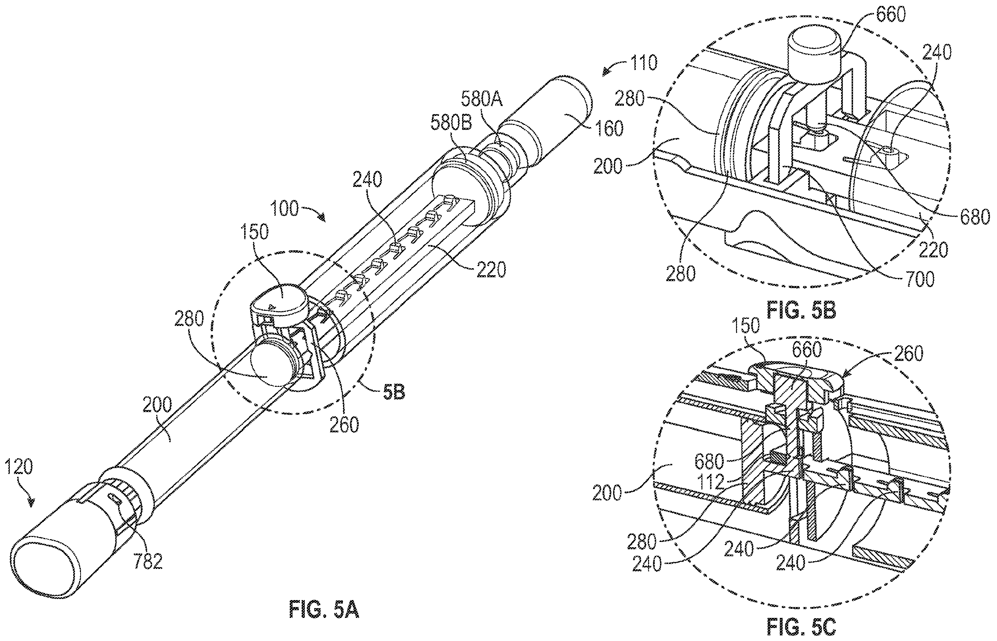

Figures 5A, 5B, and 5C illustrate in more detail the interaction between the trigger actuator 260 and the linear actuator 220. As shown in Figure 5B, the trigger actuator 260 is illustrated with the trigger cap 150 removed. The trigger actuator 260 includes a trigger button 660 that actuates movement of a trigger depressor 680 as guided by one or more trigger guides 700. The trigger depressor 680 engages with the actuator notches 240 of the linear actuator 220. When the first chamber 600 is pressurized by the pressurized gas within the cartridge 160, the linear actuator 220 is advanced toward the second end 120. Advancement of the linear actuator 220 is impeded by the contact between the trigger depressor 680 and the actuator notches 240. When the trigger cap 150 is depressed, the trigger actuator 260 and the trigger depressor 680 presses down the actuator notch 240 and releases the linear actuator 220 to advance until it engages the next actuator notch 240. When the linear actuator 220 is advanced, the plunger 280 is advanced within the medication chamber 200, and medication is conveyed through the notches (and filters if included) to be dispensed to the patient.

The nebulizer 100 includes a stopper 112 for impeding advancement of the linear actuator 220, as shown in Figure 5C. The stopper 112 can resist advancement of the linear actuator 220 in a direction toward the second end 120 when an actuator notch of the actuator notches 240 engages against the stopper 112. When the trigger cap 150 is depressed, the trigger depressor 680 of the trigger actuator 260 presses down the respective actuator notch 240 that is engaged against the stopper 112 such that the actuator notch 240 can move past the stopper 112.

In some embodiments of the present disclosure, the stopper 112 is a portion of the nebulizer 100 that extends along or adjacent to a top surface of the linear actuator 220, and the actuator notches 240 extend in a direction away from the top surface of the linear actuator. The stopper 112 extends into a pathway defined by the actuator notches 240 when the linear actuator 220 is advanced. The trigger cap 150 is configured so that, when the trigger cap 150 is depressed, the trigger depressor 680 engages against and moves the actuator notch 240 in a direction that is down or away from the stopper 112. Movement of the actuator notch 240 down or away from the stopper 112 permits the linear actuator 220 to advance under the stopper 112 and toward the second end 120.

In some embodiments of the present disclosure, the trigger may be depressed continuously creating an on-demand aerosol delivery mode in which the trigger will allow multiple doses of medication to be delivered until the user depresses the trigger button again. When the user depresses the trigger, the device will continue delivery of medication until reaching the next actuator notches on the linear actuator. In some embodiments of the present disclosure, the nebulizer can remain in the on-demand aerosol delivery mode until the user releases the trigger button.

It should be understood that the present disclosure contemplates embodiments in which the stopper 112 and actuator notches 240 are configured to move relative to each other in any direction. By way of non-limiting example, the stopper 112 and the actuator notches 240 can be positioned so that the trigger depressor 680 engages against and moves the actuator notch 240 in a lateral direction relative to the stopper 112. In some embodiments of the present disclosure, the linear actuator 220 can be biased or flexed by engagement of the trigger depressor 680 to permit advancement of the linear actuator 220 relative to the stopper 112.

Advancement of the linear actuator 220 can continue without engaging the sequential actuator notches 240 by keeping the trigger depressor 680 pressed. This action allows for extended or continuous activation for longer treatments or greater doses if desired by the user.

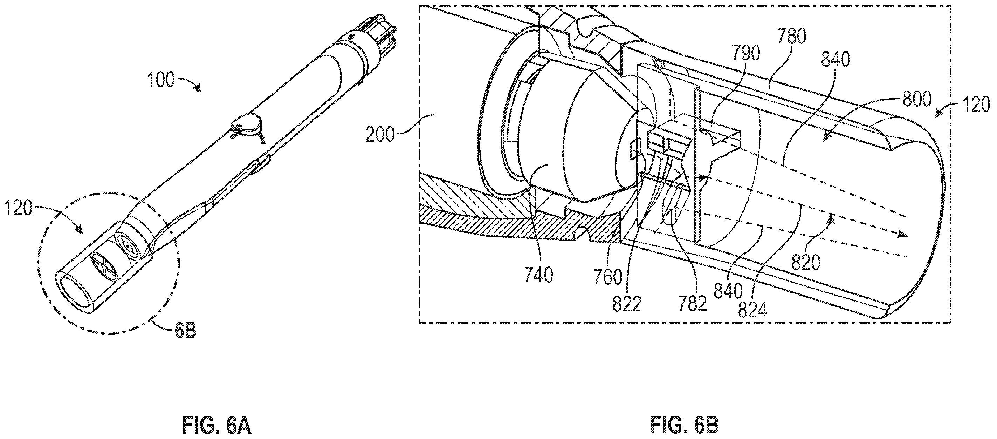

Figures 6A and 6B illustrate how the medication is conveyed to the patient when the nebulizer 100 is activated and actuated. When the linear actuator 220 is advanced and the plunger 280 is advanced within the medication chamber 200, the medication is pressurized and advanced through a nozzle housing 740 and through a nozzle 760. The second end 120 of the nebulizer 100 includes a mixing chamber housing 780 that forms a mixing chamber 800. As the medication is advanced through the nozzle 760, it is directed along an aerosolized medication flow path 820. Ambient air is entrained through openings in the mixing chamber housing 780 and follow (either by natural flow dynamics or by baffles) along an entrainment air flow path 840. The aerosolized medication flow path 820 and the entrainment air flow path 840 convey medication and air to the patient as the flow paths 820, 840 exit the second end 120.

In some embodiments, the nebulizer 100 includes a baffle 790 configured to divert or redirect the aerosolized medication flow path 820. The baffle 790 can be positioned in the mixing chamber 800 and adjacent to the nozzle 760. As the pressurized medication exits the nozzle 760, the pressurized medication moves toward the baffle 790 and is then directed around the baffle and toward the second end 120 of the nebulizer. A first portion 822 of the aerosolized medication flow path can be defined between the nozzle 760 and the baffle 790, a second portion 824 of the aerosolized medication flow path can be defined between the baffle 790 and the second end 120 of the nebulizer.

To permit entrainment of ambient air into the mixing chamber 800, the mixing chamber housing 780 can include one or more opening 782 that extends through the mixing chamber housing 780. In some embodiment of the present disclosure, the baffle 790 forms a portion of the entrainment air flow path 840 from the opening 782 into the mixing chamber 800. In some examples, a plurality of openings 782 that are spaced apart from each other and are positioned radially outward from the nozzle 760.

The aerosolized medication flow path 820 and the entrainment air flow path 840 provide for optimization of aerosol delivery, by controlling the particle size and distribution of the automized medication. It can furthermore prevent the formation of liquid medication from the aerosolized medication. Medication that changes from aerosolized to liquid phase during the start or stopping of the aerosol creation sequence can be evacuated from the mixing chamber 800 or redirected away from the second end 120 of the nebulizer to prevent the liquid medication from being consumed by the patient.

To evacuate or redirect liquid medication away from the patient, the nebulizer 100 can include a port to drain the liquid medication out of the nebulizer 100. In some embodiments of the present disclosure, the liquid medication can drain or exit the nebulizer through the opening 782.

The nebulizer 100 creates a plume of aerosol of specific MMAD/GSD for the specific liquid medication within the liquid medication vial (or the medication chamber 200) for a defined volume and/or duration for each singular dose and may be refined to a specific MMAD/GSD target by baffles in the mixing chamber housing 780 or mouthpiece. The patient inhales with a slow deep breath when the aerosol is present. As explained, entrainment air is pulled in through openings or vents and directed centrally within the mixing chamber housing flow paths to maximize the delivered dose of aerosolized medication.

Baffles in the mixing chamber housing 780 or mouthpiece can further refine the MMAD/GSD and direct any liquid medication away from the patient. The primary goal is to deliver aerosol particles in the range of about 1 to 5 microns for optimal deposition in the lower airways and prevent or limit ingestion of any condensed, liquid medication. Noting that some medications may require a different range of particle size and/or particle size distribution for optimal deposition and the device can be tuned accordingly.

The present disclosure is provided to enable any person skilled in the art to practice the various aspects described herein. The disclosure provides various examples of the subject technology, and the subject technology is not limited to these examples. Various modifications to these aspects will be readily apparent to those skilled in the art, and the generic principles defined herein may be applied to other aspects.

A reference to an element in the singular is not intended to mean "one and only one" unless specifically so stated, but rather "one or more." Unless specifically stated otherwise, the term "some" refers to one or more. Pronouns in the masculine (e.g., his) include the feminine and neuter gender (e.g., her and its) and vice versa. Headings and subheadings, if any, are used for convenience only and do not limit the invention.

The word "exemplary" is used herein to mean "serving as an example or illustration." Any aspect or design described herein as "exemplary" is not necessarily to be construed as preferred or advantageous over other aspects or designs. In one aspect, various alternative configurations and operations described herein may be considered to be at least equivalent.

As used herein, the phrase "at least one of" preceding a series of items, with the term "or" to separate any of the items, modifies the list as a whole, rather than each item of the list. The phrase "at least one of" does not require selection of at least one item; rather, the phrase allows a meaning that includes at least one of any one of the items, and/or at least one of any combination of the items, and/or at least one of each of the items. By way of example, the phrase "at least one of A, B, or C" may refer to: only A, only B, or only C; or any combination of A, B, and C.

A phrase such as an "aspect" does not imply that such aspect is essential to the subject technology or that such aspect applies to all configurations of the subject technology. A disclosure relating to an aspect may apply to all configurations, or one or more configurations. An aspect may provide one or more examples. A phrase such as an aspect may refer to one or more aspects and vice versa. A phrase such as an "embodiment" does not imply that such embodiment is essential to the subject technology or that such embodiment applies to all configurations of the subject technology. A disclosure relating to an embodiment may apply to all embodiments, or one or more embodiments. An embodiment may provide one or more examples. A phrase such an embodiment may refer to one or more embodiments and vice versa. A phrase such as a "configuration" does not imply that such configuration is essential to the subject technology or that such configuration applies to all configurations of the subject technology. A disclosure relating to a configuration may apply to all configurations, or one or more configurations. A configuration may provide one or more examples. A phrase such a configuration may refer to one or more configurations and vice versa.

In one aspect, unless otherwise stated, all measurements, values, ratings, positions, magnitudes, sizes, and other specifications that are set forth in this specification, including in the claims that follow, are approximate, not exact. In one aspect, they are intended to have a reasonable range that is consistent with the functions to which they relate and with what is customary in the art to which they pertain.

It is understood that the specific order or hierarchy of steps, or operations in the processes or methods disclosed are illustrations of exemplary approaches. Based upon implementation preferences or scenarios, it is understood that the specific order or hierarchy of steps, operations or processes may be rearranged. Some of the steps, operations or processes may be performed simultaneously. In some implementation preferences or scenarios, certain operations may or may not be performed. Some or all of the steps, operations, or processes may be performed automatically, without the intervention of a user.

The Title, Background, Summary, Brief Description of the Drawings and Abstract of the disclosure are provided as illustrative examples of the disclosure, not as restrictive descriptions. It is submitted with the understanding that they will not be used to limit the scope or meaning of the claims. In addition, in the Detailed Description, it can be seen that the description provides illustrative examples and the various features are grouped together in various embodiments for the purpose of streamlining the disclosure. This method of disclosure is not to be interpreted as reflecting an intention that the claimed subject matter requires more features than are expressly recited in each claim. Rather, as the following claims reflect, inventive subject matter lies in less than all features of a single disclosed configuration or operation. The invention is defined by the claims which follow.

## Claims

1. A pressurized gas nebulizer assembly, comprising:
a nebulizer housing comprising a first end (110) and a second end (120);
a cartridge (160) containing pressurized gas coupled to the nebulizer housing;
a linear actuator (220) comprising a series of linear positioned abutments, the linear actuator (220) configured to be movable along the nebulizer housing;
a trigger configured to engage one or more abutment of the series of linear positioned abutments as the linear actuator (220) moves along the nebulizer housing;
a medication chamber (200) configured to contain medication to be dispensed from the first (110) or second end (120) of the nebulizer housing; and
a nozzle (760), through which the medication is aerosolized before the medication is dispensed from the nebulizer housing;
wherein when pressurized gas is conveyed from the cartridge (160), pressure within the nebulizer housing from the pressurized gas forces the linear actuator (220) to move within the nebulizer housing.

2. The pressurized gas nebulizer (100) of Claim 1, wherein advancement of the linear actuator (220) is sequentially stopped at each respective abutment.

3. The pressurized gas nebulizer (100) of Claim 1, wherein advancement of the linear actuator (220) continues through a series of linear positioned abutments when the trigger is continuously depressed.

4. The pressurized gas nebulizer (100) of Claim 1, wherein pressurized gas within the cartridge (160) is not released from the cartridge (160) until the cartridge (160) is punctured.

5. The pressurized gas nebulizer (100) of Claim 4, wherein the cartridge (160) is punctured by rotation of a housing containing the cartridge (160).

6. The pressurized gas nebulizer (100) of Claim 1, further comprising a lock on the trigger to restrict actuation of the trigger until the trigger is unlocked.

7. The pressurized gas nebulizer (100) of Claim 1, wherein the nebulizer housing comprises a stopper (112) configured to be engaged against by an abutment of the series of linear positioned abutments to resist movement of the linear actuator (220) along the nebulizer housing.

8. The pressurized gas nebulizer (100) of Claim 7, wherein the trigger is engaged against the abutment of the series of linear positioned abutments, and, when the trigger is depressed, the abutment of the series of linear positioned abutments is moved away from the stopper.

9. The pressurized gas nebulizer (100) of Claim 1, further comprising a baffle (790) configured to redirect a flow of medication from the nozzle (760).

10. The pressurized gas nebulizer (100) of Claim 1, wherein the nebulizer housing comprises an opening (782) configured to permit ambient air to move into the nebulizer housing and entrain with a flow of medication from the nozzle (760).

11. A pressurized gas nebulizer (100), comprising:
a nebulizer housing;
a pressure chamber (180) for receiving a pressurized gas;
a linear actuator (220) comprising a first portion forming a portion of the pressure chamber (180). a second portion comprising a series of linear positioned abutments, and a third portion forming at least a portion of a medication chamber (200);
a trigger that is movable relative to the linear actuator (220); and
a nozzle (760) fluidly coupled to the medication chamber (200);
wherein an abutment of the series of linear positioned abutments is engaged against a portion of the nebulizer housing to resist movement of the linear actuator (220) toward medication chamber (200), and movement of the trigger moves the abutment of the series of linear positioned abutments away from the portion of the nebulizer housing to permit the linear actuator (220) to move toward the medication chamber (200) and direct a medication from the medication chamber (200) through the nozzle (760).

12. The pressurized gas nebulizer (100) of Claim 11, wherein advancement of the linear actuator (220) is sequentially stopped at each respective abutment.

13. The pressurized gas nebulizer (100) of Claim 11, further comprising a baffle (790) configured to redirect a flow of medication from the nozzle (760).

14. The pressurized gas nebulizer (100) of Claim 11, wherein advancement of the linear actuator (220) continues through a series of linear positioned abutments when the trigger is continuously depressed.

## Patentansprüche

1. Druckgasvernebleranordnung, aufweisend:
ein Verneblergehäuse mit einem ersten Ende (110) und einem zweiten Ende (120);
eine Kartusche (160), die Druckgas enthält und mit dem Verneblergehäuse gekoppelt ist;
einen Linearantrieb (220), der eine Reihe linear angeordneter Anschläge aufweist,
wobei der Linearantrieb (220) eingerichtet ist, entlang des Verneblergehäuses beweglich zu sein;
einen Auslöser, der eingerichtett ist, mit einem oder mehreren Anschlägen der Reihe der linear angeordneten Anschläge in Eingriff zu kommen, wenn sich der Linearantrieb (220) entlang des Verneblergehäuses bewegt;
eine Medikamentenkammer (200), die eingerichtet ist, ein aus dem ersten (110) oder zweiten Ende (120) des Verneblergehäuses abzugebendes Medikament aufzunehmen; und
eine Düse (760), durch die das Medikament aerosolisiert wird, bevor es aus dem Verneblergehäuse abgegeben wird;
wobei, wenn Druckgas aus der Kartusche (160) zugeführt wird, der Druck im Verneblergehäuse durch das Druckgas den Linearantrieb (220) veranlassst, sich innerhalb des Verneblergehäuses zu bewegen.

2. Druckgasvernebler (100) nach Anspruch 1, wobei der Vorschub des Linearantriebs (220) an jedem jeweiligen Anschlag sequenziell gestoppt wird.

3. Druckgasvernebler (100) nach Anspruch 1, wobei der Vorschub des Linearantriebs (220) durch eine Reihe linear angeordneter Anschläge hindurch fortgesetzt wird, wenn der Auslöser kontinuierlich betätigt wird.

4. Druckgasvernebler (100) nach Anspruch 1, wobei Druckgas innerhalb der Kartusche (160) nicht aus der Kartusche (160) freigesetzt wird, bevor die Kartusche (160) perforiert wird.

5. Druckgasvernebler (100) nach Anspruch 4, wobei die Kartusche (160) durch Rotation eines die Kartusche (160) enthaltenden Gehäuses perforiert wird.

6. Druckgasvernebler (100) nach Anspruch 1, ferner aufweisend eine Verriegelung am Auslöser, um die Betätigung des Auslösers zu verhindern, bis der Auslöser entriegelt ist.

7. Druckgasvernebler (100) nach Anspruch 1, wobei das Verneblergehäuse einen Stopper (112) aufweist, der so eingerichtet ist, dass ein Anschlag der Reihe der linear angeordneten Anschläge gegen ihn in Eingriff kommt, um eine Bewegung des Linearantriebs (220) entlang des Verneblergehäuses zu hemmen.

8. Druckgasvernebler (100) nach Anspruch 7, wobei der Auslöser gegen den Anschlag der Reihe der linear angeordneten Anschläge in Eingriff steht und, wenn der Auslöser betätigt wird, der Anschlag der Reihe der linear angeordneten Anschläge vom Stopper wegbewegt wird.

9. Druckgasvernebler (100) nach Anspruch 1, ferner aufweisend eine Ablenkplatte (790), die eingerichtet ist, einen Medikamentenstrom aus der Düse (760) umzulenken.

10. Druckgasvernebler (100) nach Anspruch 1, wobei das Verneblergehäuse eine Öffnung (782) aufweist, die eingerichtet ist, Umgebungsluft in das Verneblergehäuse eintreten zu lassen, damit sie sich mit einem Medikamentenstrom aus der Düse (760) vermischt.

11. Druckgasvernebler (100), aufweisend:
ein Verneblergehäuse;
eine Druckkammer (180) zum Aufnehmen von Druckgas;
einen Linearantrieb (220), der einen ersten Abschnitt aufweist, der einen Teil der Druckkammer (180) bildet, einen zweiten Abschnitt, der eine Reihe linear angeordneter Anschläge aufweist, und einen dritten Abschnitt, der zumindest einen Teil einer Medikamentenkammer (200) bildet;
einen Auslöser, der relativ zum Linearantrieb (220) beweglich ist; und
eine Düse (760), die fluidisch mit der Medikamentenkammer (200) gekoppelt ist;
wobei ein Anschlag der Reihe der linear angeordneten Anschläge gegen einen Abschnitt des Verneblergehäuses in Eingriff steht, um eine Bewegung des Linearantriebs (220) in Richtung der Medikamentenkammer (200) zu hemmen, und
wobei eine Bewegung des Auslösers den Anschlag der Reihe der linear angeordneten Anschläge von dem Abschnitt des Verneblergehäuses wegbewegt, um dem Linearantrieb (220) zu erlauben, sich in Richtung der Medikamentenkammer (200) zu bewegen und ein Medikament aus der Medikamentenkammer (200) durch die Düse (760) auszuleiten.

12. Druckgasvernebler (100) nach Anspruch 11, wobei der Vorschub des Linearantriebs (220) an jedem jeweiligen Anschlag sequenziell gestoppt wird.

13. Druckgasvernebler (100) nach Anspruch 11, ferner aufweisend eine Ablenkplatte (790), die eingerichtet ist, einen Medikamentenstrom aus der Düse (760) umzulenken.

14. Druckgasvernebler (100) nach Anspruch 11, wobei der Vorschub des Linearantriebs (220) durch eine Reihe linear angeordneter Anschläge hindurch fortgesetzt wird, wenn der Auslöser kontinuierlich betätigt wird.

## Revendications

1. Ensemble de nébuliseur à gaz sous pression, comprenant :
un logement de nébuliseur comprenant une première extrémité (110) et une deuxième extrémité (120) ;
une cartouche (160) contenant du gaz sous pression couplé au logement du nébuliseur ;
un actionneur linéaire (220) comprenant une série de butées positionnées linéairement, l'actionneur linéaire (220) étant configuré pour être mobile le long du logement du nébuliseur ;
un déclencheur configuré pour être en prise avec une ou plusieurs butées de la série de butées positionnées linéairement lorsque l'actionneur linéaire (220) se déplace le long du logement du nébuliseur ;
une chambre de médicament (200) configurée pour contenir un médicament à délivrer à partir de la première (110) ou de la deuxième extrémité (120) du logement du nébuliseur ; et
une buse (760), à travers laquelle le médicament est produit sous forme d'aérosol avant d'être distribué à partir du logement du nébuliseur ;
dans lequel lorsque du gaz sous pression est acheminé depuis la cartouche (160), la pression à l'intérieur du logement du nébuliseur induite par le gaz sous pression oblige l'actionneur linéaire (220) à se déplacer à l'intérieur du logement du nébuliseur.

2. Nébuliseur à gaz sous pression (100) selon la revendication 1, dans lequel l'avancement de l'actionneur linéaire (220) est séquentiellement arrêté au niveau de chaque butée respective.

3. Nébuliseur à gaz sous pression (100) selon la revendication 1, dans lequel l'avancement de l'actionneur linéaire (220) se poursuit en passant par une série de butées positionnées linéairement lorsque le déclencheur est enfoncé en continu.

4. Nébuliseur à gaz sous pression (100) selon la revendication 1, dans lequel le gaz sous pression à l'intérieur de la cartouche (160) n'est pas libéré de la cartouche (160) avant perforation de la cartouche (160).

5. Nébuliseur à gaz sous pression (100) selon la revendication 4, dans lequel la cartouche (160) est perforée par rotation d'un logement contenant la cartouche (160).

6. Nébuliseur à gaz sous pression (100) selon la revendication 1, comprenant en outre un élément de blocage sur le déclencheur pour restreindre l'actionnement du déclencheur jusqu'au déverrouillage du déclencheur.

7. Nébuliseur à gaz sous pression (100) selon la revendication 1, dans lequel le logement du nébuliseur comprend un élément d'arrêt (112) configuré pour venir en prise contre une butée de la série de butées positionnées linéairement afin de résister au mouvement de l'actionneur linéaire (220) le long du logement du nébuliseur.

8. Nébuliseur à gaz sous pression (100) selon la revendication 7, dans lequel le déclencheur est en prise contre la butée de la série de butées positionnées linéairement, et, lorsque le déclencheur est enfoncé, la butée de la série de butées positionnées linéairement est éloignée de l'élément d'arrêt.

9. Nébuliseur à gaz sous pression (100) selon la revendication 1, comprenant en outre un déflecteur (790) configuré pour rediriger un flux de médicament depuis la buse (760).

10. Nébuliseur à gaz sous pression (100) selon la revendication 1, dans lequel le logement du nébuliseur comprend une ouverture (782) configurée pour permettre à l'air ambiant de se déplacer dans le logement du nébuliseur et d'être entraîné avec un flux de médicament depuis la buse (760).

11. Nébuliseur à gaz sous pression (100), comprenant :
un logement de nébuliseur ;
une chambre de pression (180) pour recevoir un gaz sous pression ;
un actionneur linéaire (220) comprenant une première partie formant une partie de la chambre de pression (180), une deuxième partie comprenant une série de butées positionnées linéairement, et une troisième partie formant au moins une partie d'une chambre de médicament (200) ;
un déclencheur qui est mobile par rapport à l'actionneur linéaire (220) ; et
une buse (760) couplée fluidiquement à la chambre de médicament (200) ;
dans lequel une butée de la série de butées positionnées linéairement est en prise contre une partie du logement du nébuliseur pour résister au mouvement de l'actionneur linéaire (220) vers la chambre de médicament (200), et le mouvement du déclencheur éloigne la butée de la série de butées positionnées linéairement de la partie du logement du nébuliseur afin de permettre à l'actionneur linéaire (220) de se déplacer vers la chambre de médicament (200) et de diriger un médicament de la chambre de médicament (200) à travers la buse (760).

12. Nébuliseur à gaz sous pression (100) selon la revendication 11, dans lequel l'avancement de l'actionneur linéaire (220) est séquentiellement arrêté au niveau de chaque butée respective.

13. Nébuliseur à gaz sous pression (100) selon la revendication 11, comprenant en outre un déflecteur (790) configuré pour rediriger un flux de médicament depuis la buse (760).

14. Nébuliseur à gaz sous pression (100) selon la revendication 11, dans lequel l'avancement de l'actionneur linéaire (220) se poursuit en passant par une série de butées positionnées linéairement lorsque le déclencheur est enfoncé en continu.
